# EUROPEAN PATENT SPECIFICATION

(11) **EP 3 840 685 B1**
(45) Date of publication and mention of the grant of the patent: **01.10.2025**
(21) Application number: 19765395.9
(22) Date of filing: 23.08.2019
(51) Int. Cl.: A61B 90/00, A61B 90/96, A61B 90/30, A61B 17/00, H04N 1/00, H04N 1/40, H04N 1/407, A61B 34/00, A61B 90/98

(54) **OFF-CAMERA CALIBRATION PARAMETERS FOR AN IMAGE CAPTURE DEVICE**
KAMERAKALIBRIERUNGSPARAMETER FÜR EINE BILDERFASSUNGSVORRICHTUNG
PARAMETRES DE CALIBRATION HORS-CAMERA POUR UN APPAREIL DE CAPTURE D'IMAGES

(30) Priority: 24.08.2018 US 201862722314 P
(43) Date of publication of application: 30.06.2021
(73) Proprietor: Intuitive Surgical Operations, Inc., Sunnyvale, CA 94086 (US)
(72) Inventor: HALDERMAN, Jonathan, Sunnyvale, California 94086 (US); HOFFMAN, Brian D., Sunnyvale, California 94086 (US); LIOU, Derek C., Sunnyvale, California 94086 (US); MCDOWALL, Ian E., Sunnyvale, California 94086 (US)
(74) Representative: MacDougall, Alan John Shaw
(86) International application number: PCT/US2019/047932
(87) International publication number: WO 2020/041717

(56) References cited:
- EP-A1- 2 811 449
- EP-A1- 2 811 449
- WO-A2-2018/089816
- WO-A2-2018/089816
- US-A1- 2014 015 947
- US-A1- 2014 029 052

## Description

### CROSS-REFERENCE TO RELATED APPLICATIONS

This application claims the benefit of U.S. Provisional Patent Application Serial No. 62/722,314 filed August 24, 2018.

### BACKGROUND

Image capture devices are typically factory calibrated before use in the field. During the calibration process, one or more images of test patters are captured and used as a reference to generate calibration data for an image capture device. For example, one or more transforms are used to adjust a captured image from the image capture device to align with a known image of test patterns. The calibration process may calibrate the captured image to correct for color, optical, alignment, or other image sensor-induced or optical-induced errors or variations in the image capture device. The calibration process generates a set of calibration data that is stored with the image capture device and applied to correct images captured by the image capture device in use.

WO2018/089816 discloses a teleoperated surgical system comprising: a first robotic surgical instrument; an image capture; a user display; a user input command device coupled to receive user input commands to control movement of the first robotic surgical instrument; and a movement controller coupled to scale a rate of movement of the first robotic surgical instrument, based at least in part upon a surgical skill level at using the first robotic surgical instrument of the user providing the received user input commands, from a rate of movement indicated by the user input commands received at the user input command device.

US 2014/029052 discloses a method and system for subscription based access to services for image systems including an image acquisition device configured to generate image data describing a target object in a target area of the image acquisition device and an image display device configured to generate a human perceptible rendering of the target object based on the image data.

US 2014/015947 discloses an endoscope system which includes an imaging apparatus that outputs, as image information, an electrical signal photoelectrically converted from a plurality of pixels, outside the imaging apparatus, and a processing apparatus that is connected to the imaging apparatus so as to bi-directionally communicate with the imaging apparatus. The imaging apparatus includes a first recording unit that records identification information for identifying the imaging apparatus. The processing apparatus includes a second recording unit that records correction information for correcting characteristics of the imaging apparatus in association with the identification information, and a control unit that acquires, from the second recording unit, the correction information corresponding to the identification information received from the imaging apparatus and transmits the correction information to the imaging apparatus.

### SUMMARY

The present invention provides a system and a method as set out in the appended independent claims. Optional features are provided in the dependent claims. A first aspect of the disclosure includes a system comprising a network accessible database configured to store calibration data for an image capture device. The system also comprises a controller system comprising an image processor configured to receive images captured by the image capture device. The image processor further configured to retrieve the calibration data from the network accessible database via a network connection. The image processor further configured to process the received images based on the retrieved calibration data.

In some implementations of the first aspect of the disclosure, the system further comprises a tool comprising the image capture device and electrically coupled to the controller system. The tool has a unique identifier.

In some implementations of the first aspect of the disclosure, the calibration data is indexed with the image capture device in the network accessible database based on the unique identifier.

In some implementations of the first aspect of the disclosure, the unique identifier is maintained by the tool, and wherein the image processor is further configured to obtain the unique identifier from the tool.

In some implementations of the first aspect of the disclosure, the unique identifier is maintained by the tool on a memory device of the tool or on a visual indicator on a surface of the tool.

In some implementations of the first aspect of the disclosure, the image processor further comprises an optical scanner configured to read the visual indicator from the surface of the tool to obtain the unique identifier from the tool.

In some implementations of the first aspect of the disclosure, the optical scanner is a camera or a bar code reader.

In some implementations of the first aspect of the disclosure, the visual indicator is printed, adhered, labeled, embossed, or stamped onto the surface of the tool.

In some implementations of the first aspect of the disclosure, the visual indicator is an alphanumeric serial number, a linear barcode that encodes the unique identifier, a twodimensional barcode that encodes the unique identifier, or image encoded with the unique identifier.

In some implementations of the first aspect of the disclosure, the tool comprises an endoscope, and the image capture device comprises a pair of image sensors configured to capture stereoscopic image data of a surgical site during a surgical procedure.

In some implementations of the first aspect of the disclosure, the calibration data comprises data for processing the received images to correct the received images based on one or more of a color of light emitted by an illuminator configured to illuminate a scene captured by the received images, calibration images captured by the image capture device, optical distortions introduced by an optical system of the image capture device, a stereoscopic image calibration, or a color space mapping.

In some implementations of the first aspect of the disclosure, the system further comprises a calibration processor in communication with the network accessible database. The calibration processor configured to receive calibration images captured by the image capture device and generate the calibration data based on applying a calibration algorithm to one or more of the calibration images.

In some implementations of the first aspect of the disclosure, the calibration images are stored in the network accessible database with the calibration data.

In some implementations of the first aspect of the disclosure, the calibration processor is configured to generate the calibration data upon receiving a request for the calibration data from the controller system.

In some implementations of the first aspect of the disclosure, the calibration data is different from a prior set of calibration data for the image capture device, wherein the prior set of calibration data is generated by the calibration processor based on applying a different calibration algorithm to one or more of the calibration images.

In some implementations of the first aspect of the disclosure, the calibration algorithm is selected based on a type of procedure in which the image capture device is to be used or based on a configuration of the controller system.

In some implementations of the first aspect of the disclosure, the calibration data is generated to enhance an image feature significant to the type of procedure in which the image capture device is to be used.

In some implementations of the first aspect of the disclosure, the image feature is one or more of a focus level, sharpness, color fidelity, or color accuracy.

In some implementations of the first aspect of the disclosure, the calibration data further adversely impacts a second image feature.

In some implementations of the first aspect of the disclosure, the configuration of the controller system comprises one or more of an illumination source provided by the controller system, a type of the image processor, or a software release version of the controller system.

In some implementations of the first aspect of the disclosure, the calibration processor is further configured to retrieve an operational image captured by the image capture device during the procedure from the controller system and to adjust the calibration data based on the operational images.

In some implementations of the first aspect of the disclosure, a luminance value of the operational image is compared to an expected luminance value determined based on a known distance to an object captured in the operational image and a known power output to an illumination source that is illuminating the object captured in the operational image.

In some implementations of the first aspect of the disclosure, a color space of the operation image is compared to an expected color space of the image capture device to determine whether a center of the color space is offset from a center of the expected color space.

In some implementations of the first aspect of the disclosure, a stereoscopic alignment of the operational image is evaluated to identify misalignment with a corresponding operational image.

In some implementations of the first aspect of the disclosure, the calibration processor is further configured to receive a user feedback survey evaluating the calibration data in comparison to the prior set of calibration data.

In some implementations of the first aspect of the disclosure, the controller system further comprises a calibration cache memory configured to store the calibration data retrieved from the network accessible database.

In some implementations of the first aspect of the disclosure, the controller system is further configured to store calibration data of image capture devices known to have been shipped to a location associated with a location of the controller system.

In some implementations of the first aspect of the disclosure, the controller system is further configured to store calibration data of a predetermined number of previously used image capture devices.

In some implementations of the first aspect of the disclosure, the controller system is further configured to store default calibration data for a type of the image capture device.

In some implementations of the first aspect of the disclosure, the controller system is further configured to compare a cached set of calibration data for the image capture device to the calibration data stored on the network accessible database prior to retrieving the calibration data from the network accessible database.

A second aspect of the disclosure includes a method comprising storing calibration data for an image capture device in a network accessible database. The method also comprises retrieving, by a controller system, the calibration data from the network accessible database via a network connection. The method also comprises receiving, by the controller system, images captured by the image capture device. The method also comprises processing, by an image processor of the controller system, the received images based on the retrieved calibration data.

A third aspect of the disclosure includes a surgical system comprising a network accessible database configured to store calibration data for an endoscopic image capture device. The surgical system also comprises an image processor configured to be communicatively coupled to the endoscopic image capture device to receive image data of a surgical site during a surgical procedure. The image processor is configured to retrieve the calibration data from the network accessible database via a network connection. The image processor further configured to process the received images based on the retrieved calibration data. The surgical system further comprises a user control system coupled to the image processor and configured to receive the processed images from the image processor. The user control system comprising a display configured to display the processed images.

In some implementations of the third aspect of the disclosure, the surgical system further comprises a manipulator system comprising a manipulator arm configured to attach to the endoscopic image capture device and configured to physically alter a pose of the endoscopic image capture device during the surgical procedure.

In some implementations of the third aspect of the disclosure, the user control system further comprises a manipulation user interface. Upon actuation of the manipulation user interface, the user control system is configured to issue commands to the manipulator system to change the pose of the endoscopic image capture device.

A fourth aspect of the disclosure includes a method of processing images of a surgical procedure captured by an endoscopic image capture device. The method comprises receiving, by an image processor, the images captured by the endoscopic image capture device. The method also comprises retrieving, by the image processor, calibration data for the endoscopic image capture device from a network accessible database. The method also comprises processing, by the image processor, the received images from the endoscopic image capture device based on the retrieved calibration data. The method also comprises transmitting, by the image processor, the processed images to a user control system. The method also comprises displaying, on the user control system, the processed images on a display. The present invention provides a system and method as set out in the independent claims. Advantageous embodiments are defined in the dependent claims.

These and other features will be more clearly understood from the following detailed description taken in conjunction with the accompanying drawings and claims.

### BRIEF DESCRIPTION OF THE DRAWINGS

For a more complete understanding of the present disclosure, reference is now made to the following brief description, taken in connection with the accompanying drawings and detailed description, wherein like reference numerals represent like parts.
FIG. 1 is a plan view of a minimally invasive teleoperated surgical system.
FIG. 2 is a perspective view of a user control system.
FIG. 3 is a perspective view of an electronics cart.
FIG. 4 is a diagrammatic illustration of a teleoperated surgical system.
FIG. 5 is a perspective view of an endoscopic image capture device.
FIG. 6 is a diagrammatic illustration of an image capture device calibration tool.
FIG. 7 is a flowchart illustrating an example process of obtaining calibration data for an image capture device.
FIG. 8 is a flowchart illustrating an example process of sending calibration data in response to a request.
FIG. 9 is a flowchart illustrating an example process of determining changed calibration parameters or maintenance conditions based on field images captured by an image capture device.
FIGS. 10A-10D are chromaticity diagrams generated from processed field images showing a shift in a center of the color space of an image capture device.
FIG. 11 is a flowchart illustrating an example process of conducting a calibration parameter survey.
FIG. 12 illustrates an exemplary computer system.

### DETAILED DESCRIPTION

It should be understood at the outset that although illustrative implementations of one or more embodiments are illustrated below, the disclosed systems and methods may be implemented using any number of techniques, whether currently known or in existence. The disclosure should in no way be limited to the illustrative implementations, drawings, and techniques illustrated below, but may be modified within the scope of the appended claims along with their full scope of equivalents. Use of the phrase "and/or" indicates that any one or any combination of a list of options can be used. For example, "A, B, and/or C" means "A", or "B", or "C", or "A and B", or "A and C", or "B and C", or "A and B and C".

Elements described in detail with reference to one embodiment, implementation, or application may, whenever practical, be included in other embodiments, implementations, or applications in which they are not specifically shown or described. For example, if an element is described in detail with reference to one embodiment and is not described with reference to a second embodiment, the element may nevertheless be claimed as included in the second embodiment. Thus, to avoid unnecessary repetition in the following description, one or more elements shown and described in association with one embodiment, implementation, or application may be incorporated into other embodiments, implementations, or aspects unless specifically described otherwise, unless the one or more elements would make an embodiment or implementation non-functional, or unless two or more of the elements provide conflicting functions.

Aspects of the invention are described primarily in terms of an implementation using a da Vinci^{®} Surgical System (specifically, a Model IS4000, marketed as the da Vinci^{®} Xi^{™} HD^{™} Surgical System), commercialized by Intuitive Surgical, Inc. of Sunnyvale, California. Knowledgeable persons will understand, however, that inventive aspects disclosed herein may be embodied and implemented in various ways, including robotic and, if applicable, non-robotic embodiments and implementations. Implementations on da Vinci^{®} Surgical Systems *(e.g.,* the Model IS4000 da Vinci^{®} Xi^{™} Surgical System, the Model IS3000 da Vinci Si^{®} Surgical System) are merely exemplary and are not to be considered as limiting the scope of the inventive aspects disclosed herein.

In accordance with various aspects, the present disclosure describes a system and method for off-camera calibration of an image capture device. Calibration data is typically maintained in a read-only memory of the image capture device and is not able to be changed or updated after the image capture device leaves the factory. Calibration data for image capture devices typically does not take into account wear of the device over time. Therefore, the calibration data stored on the image capture device may become less accurate over time and result in processed images from the image capture device with more errors.

For example, an endoscopic image capture device includes a camera housing that comprises a flexible cable with a fiber optic bundle. The cable comprises a connector configured to couple the fiber optic bundle to a light source. The camera housing also comprises a read-only memory that stores calibration data for the endoscopic image capture device. The camera housing also comprises a rigid camera shaft with a camera tip on a distal end. The camera tip includes one or more image sensors and associated optics systems. The camera shaft also comprises a second fiber optic bundle configured to convey light received from the fiber optic bundle in the flexible cable to the camera tip to illuminate a scene being imaged by the one or more image sensors, such as a diagnostic or surgical procedure. Images captured by the one or more image sensors in the camera tip are conveyed via a wired or wireless electrical connection to the camera housing and in turn conveyed via a wired or wireless electrical connection in the flexible cable to the connector.

A controller system comprises a socket configured to accept the connector. The controller system comprises a light source coupled to the socket and configured to supply light to the fiber optic bundle in the flexible cable. The controller system also comprises an image processor coupled to socket and configured to receive the images conveyed via the electrical connection in the flexible cable. The image processor is configured to read the calibration data from the read-only memory in the camera housing upon the connector being inserted to the socket. The image processor processes the received images based on the read calibration data to generate one or more processed images. As the endoscopic image capture device is used, the optical transmission properties of the fiber optic bundle in the flexible cable may change over time. Additionally, the camera tip may wear over time due to use in medical procedures. Therefore, at least the optical properties of the endoscopic image capture device will change over time, resulting in calibration data that is not optimized to the current condition of the endoscopic image capture device.

Additionally, the endoscopic image capture device may be coupled to different controller systems at different times, where one or more of the controller systems may have a different configuration. Different controller systems may have different system configurations, such as different image processors, vision systems, or other hardware variations, different software release versions, and/or different light sources with different spectral responses. Therefore, the calibration data may not be optimized for the particular controller system to which the endoscopic image capture device is currently coupled.

Moreover, advances in the transforms used to generate the calibration data from the captured test patterns are not able to be retroactively applied to existing surgical endoscopic image capture devices. Accordingly, the calibration data stored on the surgical endoscopic image capture device may not be optimized according to current knowledge and technology.

In various implementations of the pending disclosure, the calibration data is moved off-camera and is obtained by the controller system from a central calibration server. Upon an image capture device, such as an endoscopic image capture device, being connected to the controller system, the controller system obtains a unique identifier of the image capture device. While the various examples provided herein are described with respect to an endoscopic image capture device, the pending disclosure is not so limited and is intended to encompass any device coupled to the controller system that has corresponding calibration data to control the operation of the device or processing of data received from the device. Likewise, the pending disclosure is intended to encompass any image capture device that is coupled to a controller system for processing images captured by the image capture device. For example, the pending disclosure may equally apply to a borescope or other such inspection camera.

The unique identifier may be maintained in a memory of the image capture device. Alternatively, a reader coupled to the controller system reads the unique identifier from the image capture device. For example, the reader may be a radio frequency identifier (RFID), Bluetooth^{®} low energy (BLE), Wi-Fi^{®}, or other wireless tag reader and the unique identifier may be stored in a wireless tag, such as an RFID, BLE, Wi-Fi^{®}, or other wireless tag.

In another example, the unique identifier is provided as an optically readable label on the housing of the image capture device. The label may include an alphanumeric string, symbol, or code in an encrypted or non-encrypted format. The label may be embossed, glued, adhered, printed, or otherwise affixed to the housing of the image capture device. The unique identifier may be read from a camera, barcode reader, or other optical scanner coupled to or otherwise in communication with the controller system.

Using the obtained unique identifier of the image capture device, the controller system obtains calibration data for the image capture device from the central calibration server. The controller system then processes images received from the image capture device based on the retrieved calibration data. In some implementations, the unique identifier is a universally unique identifier (UUID) for a medical device.

By off-loading the calibration data from the image capture device, the memory requirements of the image capture device may be reduced, leading to less expensive image capture devices. For example, newly produced image capture devices may omit the read-only calibration memory or otherwise reduce the size of the read-only memory to only store the unique identifier of the image capture device.

The calibration data may be periodically or dynamically generated at the central calibration server. Therefore, the methodology for calculating one or more of the calibration parameters may be changed based on the type of procedure to be performed, based on the current equipment configuration, and/or based on improvements to the calibration methodology. Moreover, obtaining the calibration data from the central calibration server extends the life and/or functionality of existing equipment in the field, which may otherwise store outdated calibration data or calibration data not optimized for a particular procedure.

Because the controller system is in regular communication with the central calibration server, additional functionality may be enabled. For example, images captured from the image capture device may be periodically communicated to the central calibration server. These field images may be processed to identify improvements to the calibration data for a particular image capture device over time or flag the image capture device for maintenance. For example, the field captured images may reveal spectral attenuation in the captured images over time due to wear of the fiber optic bundle in the flexible cable. The spectral attenuation may be corrected by adjustments to the calibration data or adjustments to the light source provided to the image capture device. Likewise, shifts in the color space of the one or more image sensors over time may be corrected by adjustments to the calibration data or adjustments to the light source provided to the image capture device. Additionally, alignment issues in stereoscopic images may be identified and flagged for maintenance.

As another example, preference studies may be conducted with end-users of the image capture device with different sets of calibration data. For example, different color mappings or distortion corrections may be used in different sets of calibration data and feedback from end-users may be obtained to inform an optimized set of calibration data. Additional controls may be added to prevent use of the controller system with non-registered image capture devices, facilitate recall control of image capture devices, and limit a total number of uses of image capture devices before requiring refurbishing.

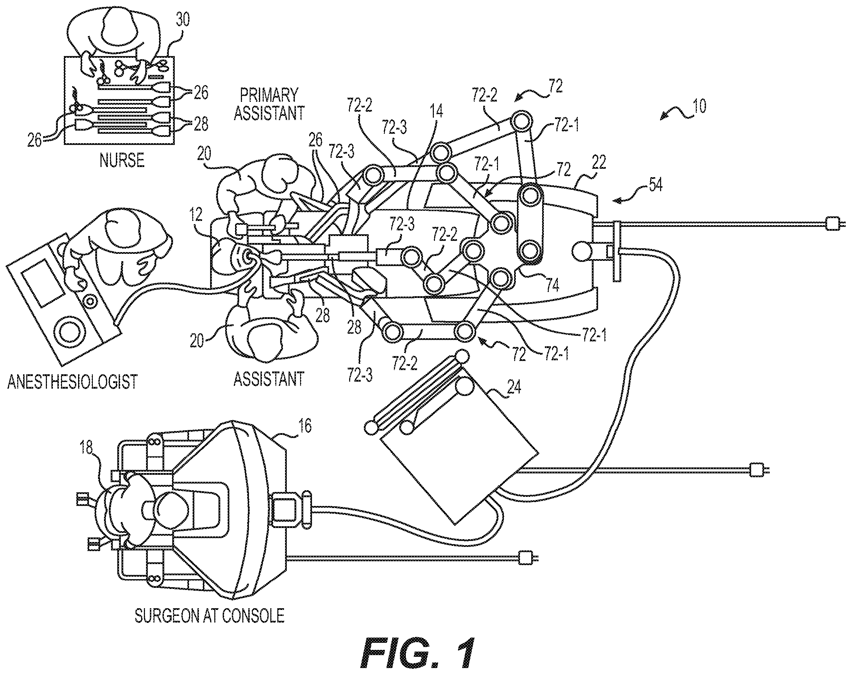

Referring now to the drawings, in which like reference numerals represent like parts throughout the several views, FIG. 1 is a plan view of a minimally invasive teleoperated surgical system 10, typically used for performing a minimally invasive diagnostic or surgical procedure on a patient 12 who is lying on a mobile operating table 14. The system includes a user control system 16, such as a mobile surgeon's console for use by a surgeon 18 during the procedure. One or more assistants 20 may also participate in the procedure. The minimally invasive teleoperated surgical system 10 further includes a manipulating system 22, such as a mobile patient-side cart, and a mobile electronics cart 24. In some embodiments, the table 14, user control system 16, manipulating system 22, and the electronics cart 24 are wheel mounted to provide mobility.

The manipulating system 22 or other such manipulating system includes multiple segmented mechanical support arms 72, each having one end portion rotatably mounted to a vertical support structure 74 and having another end mounting a removably coupled surgical instrument 26. In some of embodiments, each mechanical support arm 72 includes a first segment 72-1, a second segment 72-2 and a third segment 72-3. During setup for a procedure, the multiple segments of at least one support arm 72 are moved to position a surgical instrument for insertion within a minimally invasive incision in the body of the patient 12.

During the procedure, while instruments are inserted within a patient's body cavity, the surgeon 18 views the surgical site through the user control system 16. An image of the surgical site can be obtained by an endoscope 28, such as a stereoscopic endoscope, which can be manipulated by the manipulating system 22 to orient the endoscope 28. Computer processor(s) located on the electronics cart 24 can be used to process the images of the surgical site for subsequent display to the surgeon 18 through the user control system 16. The computer processor(s) may alternatively be referred to herein as an image processor or video processor.

One or more illumination sources or illuminators may also be provided on the electronics cart 24 to provide light for use by the endoscope 28 for illuminating the surgical site. The illuminators may include a white light source, a colored light source (e.g., red, green, blue, cyan, magenta, yellow, etc.), an infrared light source, a laser light source, or any other type of light source or combination thereof. Different illuminators may be used at different points in time in a surgical or diagnostic procedure. For example, the electronics cart 24 may be controlled, such as through a selection on the user control system 16, to provide light from a first set of one or more of the illuminators at a first time and provide light from a second set of one or more of the illuminators at a second time.

The number of surgical instruments 26 used at one time will generally depend on the diagnostic or surgical procedure and the space constraints within the operating room among other factors. If it is necessary to change one or more of the surgical instruments 26 being used during a procedure, an assistant 20 can remove the surgical instrument 26 from the manipulating system 22, and replace it with another surgical instrument 26 from a tray 30 in the operating room.

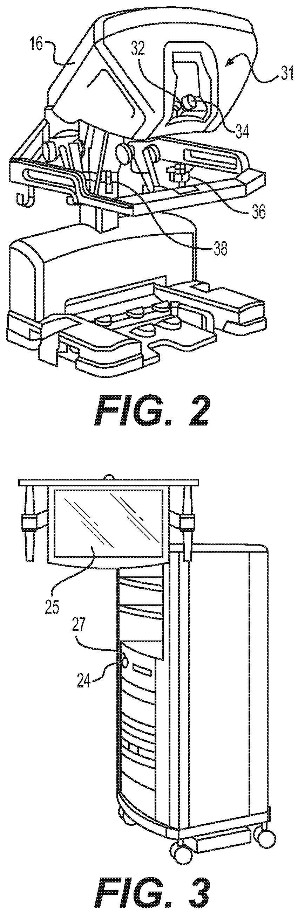

FIG. 2 is a perspective view of the user control system 16. The user control system 16 includes a display area 31 with a left eye display 32 and a right eye display 34 for presenting the surgeon 18 with a coordinated stereoscopic view of the surgical site that enables depth perception.

The console 16 further includes one or more control inputs 36. One or more surgical instruments installed for use on the manipulating system 22 (shown in FIG. 1) move in response to surgeon 18's manipulation of the one or more control inputs 36. The control inputs 36 can provide the same mechanical degrees of freedom as their associated surgical instruments 26 (shown in FIG. 1) to provide the surgeon 18 with telepresence, or the perception that the control inputs 36 are integral with the instruments 26 so that the surgeon has a strong sense of directly controlling the instruments 26. To this end, position, force, and tactile feedback sensors (not shown) may be employed to transmit position, force, and tactile sensations from the surgical instruments 26 back to the surgeon's hands through the control inputs 36. A height of the control inputs 36 may be adjusted with a height adjustment lever 38.

The user control system 16 is usually located in the same room as the patient so that the surgeon can directly monitor the procedure, be physically present if necessary, and speak to a patient-side assistant directly rather than over the telephone or other communication medium. But, the surgeon can be located in a different room, a completely different building, or other remote location from the patient allowing for remote surgical procedures.

FIG. 3 is a perspective view of the electronics cart 24. The electronics cart 24 can be coupled with the endoscope 28 and includes a computer processor to process captured images for subsequent display, such as to a surgeon on the user control system 16, or on another suitable display located locally and/or remotely. For example, if a stereoscopic endoscope is used, a computer processor on electronics cart 24 can process the captured images to present the surgeon with coordinated stereo images of the surgical site. Such coordination can include alignment between the opposing images and can include adjusting the stereo working distance of the stereoscopic endoscope.

As another example, image processing can include the use of previously or dynamically determined camera calibration parameters to compensate for imaging errors of the image capture device, such as optical aberrations. The calibration data may be obtained by the electronics cart 24 from a central calibration server as discussed in more detail below.

Because the calibration data is obtained from the central calibration server instead of from the image capture device itself, new calibration data may be used with existing image capture devices that may store current calibration data on a local memory of the image capture device. Therefore, the useful life or functionality of the existing image capture devices may be extended by remotely obtaining calibration data for the existing image capture device. Likewise, the memory requirements of new image capture devices may be reduced, leading to less expensive image capture devices. For example, newly produced image capture devices may omit a memory for storing calibration data or otherwise reduce the size of the memory to only store a unique identifier of the image capture device. Additionally, the methodology for calculating one or more of the calibration parameters may be changed based on the type of procedure to be performed, based on the current equipment configuration, and/or based on improvements to the calibration methodology.

The electronics cart 24 may also maintain the obtained calibration data in a local cache. In addition to maintaining the obtained calibration data in the local cache, the electronics cart 24 may also maintain calibration data for one or more image capture devices anticipated to be used with the electronics cart 24. For example, upon a new image capture device being ordered, delivered, or received at a location with the electronics cart 24, the electronics cart 24 may obtain or receive calibration data for the new image capture device. In some implementations, the central calibration server may push the calibration data for the new image capture device to be maintained on the local cache of the electronics cart 24. The local cache of the electronics cart 24 may also maintain a default set of calibration data for use when there is limited or no connectivity with the central calibration server.

Optionally, equipment in electronics cart 24 may be integrated into the user control system 16 or the manipulating system 22, or it may be distributed in various other locations in the operating room. More generally, the electronics cart 24 or user control system 16 with the integrated equipment from the electronics cart 24 may be referred to herein as a controller system for obtaining calibration data and processing images from the image capture device based on the retrieved calibration data.

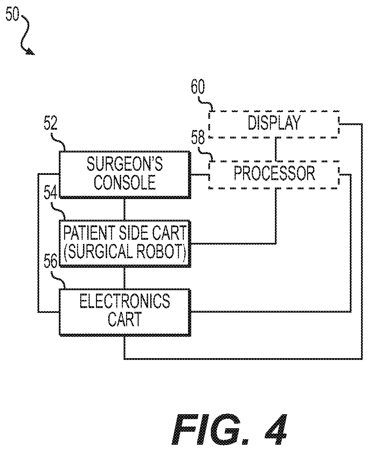

FIG. 4 diagrammatically illustrates a teleoperated surgical system 50 (such as the minimally invasive teleoperated surgical system 10 of FIG. 1). A user control system 52 (such as user control system 16 in FIG. 1) can be used by a surgeon to control a manipulating system 54 (such as manipulating system 22 in FIG. 1) during a minimally invasive procedure. The manipulating system 54 can use an image capture device, such as a stereoscopic endoscope, to capture images of a surgical site and output the captured images to a computer processor located on an electronics cart 56 (such as the electronics cart 24 in FIG. 1). The computer processor typically includes one or more data processing boards purposed for executing computer readable code stored in a non-volatile memory device of the computer processor. As with the electronics cart 24, the electronics cart 56 also includes one or more illumination sources for supplying light to the image capture device. The electronics cart 56 may also comprise a calibration cache for maintaining a local cache of calibration data similar to the electronics cart 24.

In one aspect, the computer processor can process the captured images in a variety of ways prior to any subsequent display. For example, the computer processor can use of previously or dynamically determined camera calibration parameters to compensate for imaging errors of the image capture device prior to displaying the processed images to the surgeon via the user control system 52. Additionally or in the alternative, the captured images can undergo image processing by a computer processor located outside of electronics cart 56. In one aspect, teleoperated surgical system 50 includes an optional computer processor 58 (as indicated by dashed line) similar to the computer processor located on electronics cart 56, and manipulating system 54 outputs the captured images to computer processor 58 for image processing prior to display on the user control system 52. In another aspect, captured images first undergo image processing by the computer processor on electronics cart 56 and then undergo additional image processing by computer processor 58 prior to display on the user control system 52. In some implementations, the electronics cart 56 and/or computer processor 58 are collectively referred to as a controller system for obtaining calibration data and processing images from the image capture device based on the retrieved calibration data. In some implementations, the controller system also supplies light to the image capture device from one or more illumination sources.

Teleoperated surgical system 50 can include an optional display 60, as indicated by dashed line. Display 60 is coupled with the computer processor located on the electronics cart 56 and with computer processor 58, and captured images processed by these computer processors can be displayed on display 60 in addition to being displayed on a display of the user control system 52. In various implementations, the display 60 may be located on the electronics cart 56, such as with a display 25 on the electronics cart 24. In some implementations, the display 60 may be separate from the user control system 52 and the electronics cart 58.

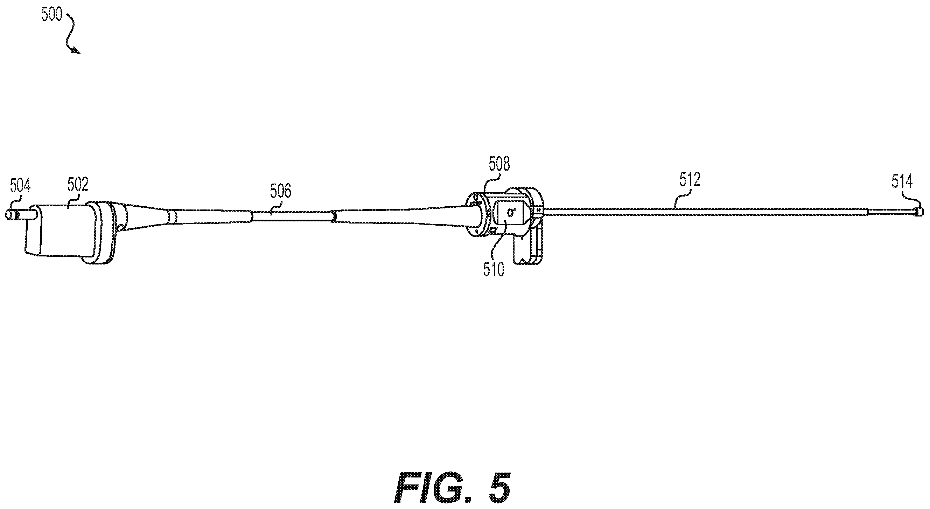

FIG. 5 is a perspective view of an endoscopic image capture device 500. The endoscopic image capture device 500 includes a connector 502, a flexible cable 506, a camera housing 508, a rigid camera shaft 5 1 2, and a camera tip 514. The connector 502 comprises an optical port 504 and an electrical port (not shown). The connector 502 is sized and shaped to be inserted into a socket on the electronics cart 56, such as in a socket 27 or other mating receptacle of the electronics cart 24. The optical port 504 is configured to receive light supplied by the electronics cart 24 to the socket 27.

The flexible cable 506 is coupled between the connector 502 and the camera housing 508. The flexible cable 506 includes a fiber optic bundle configured to convey light received from the optical port 504 of the connector 502 to the camera housing 508. The flexible cable 506 also includes an electrical connection configured to provide electrical communication between the electrical port of the connector 502 and the camera housing 508. The electrical connection may be a wired or wireless connection. In some implementations, the wired connection is a wire, ladder line, twisted wire pair, universal serial bus (USB) cable, Ethernet cable, or other wired communication line.

The camera housing 508 receives the distal end of the fiber optic bundle from the flexible cable 506. The camera housing 508 also receives a proximal end of the rigid camera shaft 512. A distal end of the rigid camera shaft 5 12 includes the camera tip 514 with one or more image sensors and associated optics systems. For example, the camera tip 514 may include two image sensors with respective optical components for capturing stereoscopic images of a scene, such as a surgical or diagnostic procedure. The rigid camera shaft 512 may also include a second fiber optic bundle configured to convey light received at the camera housing 508 from the fiber optic bundle in the flexible cable to the camera tip 514 to illuminate a scene being imaged by the one or more image sensors.

The rigid camera shaft 512 may also include a second electrical connection configured to provide electrical communication between the one or more image sensors of the camera tip 514 and the camera housing 508. Images captured by the one or more image sensors in the camera tip 514 are conveyed via the electrical connection in the rigid camera shaft 512 to the camera housing 508. The electrical connection may be a wired or wireless connection. In some implementations, the wired connection is a wire, ladder line, twisted wire pair, universal serial bus (USB) cable, Ethernet cable, or other wired communication line.

The camera housing 508 may also comprise one or more camera control units (not shown) configured to supply power and provide control signals for capturing images from the one or more image sensors in the camera tip 514. For example, when the camera tip 514 comprises two image sensors for capturing stereoscopic images, the camera housing 508 may have a respective camera control unit for controlling each of the two image sensors. The one or more camera control units are also configured to communicate the captured images to the electrical port of the connector 502 for processing by the electronics cart 56 and/or computer processor 58.

The camera housing 508 may also comprise a display 510 for displaying one or more operational controls of the endoscopic image capture device 500.

The camera housing 508 may also comprises a read-only memory (not shown) that stores a unique identifier of the endoscopic image capture device 500. In some implementations, the unique identifier is a universally unique identifier (UUID) for a medical device. The read-only memory does not store calibration data for the endoscopic image capture device 500. The read-only memory is smaller and cheaper than the read-only memory used in prior endoscopic image capture devices that additionally stored calibration data.

In some implementations, the read-only memory in the camera housing 508 may be omitted entirely. Instead a wireless identification tag installed on or in the camera housing 508 or an optically readable label on an exterior surface of the camera housing 508 may provide the unique identifier of the endoscopic image capture device 500. For example, the wireless identification tag may be an RFID, BLE, Wi-Fi^{®}, or other wireless tag. The label may include an alphanumeric string, symbol, or code in an encrypted or non-encrypted format. The label may be embossed, glued, adhered, printed, or otherwise affixed to the camera housing 508.

When an optically readable label is used to provide the unique identifier on the camera housing 508, an optical scanner coupled to or otherwise in communication with the controller system is used to read the label to obtain the unique identifier. The optical scanner may be a camera, barcode reader, or other optical scanner. For example, any of the manipulating system 54, the electronics cart 56, and/or the computer processor 58 may comprise an optical scanner configured to read the label on the camera housing 508 to obtain the unique identifier of the endoscopic image capture device 500.

In one implementation, the manipulating system 54 comprises a camera (not shown) configured to capture an image of the camera housing 508 of the endoscopic image capture device 500 when installed thereon. The image captured by the camera on the manipulating system 54 is processed by the electronics cart 56 and/or computer processor 58 to identify the unique identifier in the captured image.

In another implementation, the socket 27 or other mating receptacle of the electronics cart 24 comprises the optical scanner. A surface of the connector 502 or the optical port 504 comprises the optically readable label and is configured to be in a field of view or otherwise face the optical scanner when the connector 502 is inserted into the socket 27. The optical scanner of the electronics cart 24 is configured to read the label to obtain the unique identifier of the endoscopic image capture device 500 upon insertion of the connector 502 into the socket 27. Therefore, a user is not required to perform any additional action to scan the unique identifier other than insert the connector 502 into the socket 27. Some users may attempt "spoofing" the electronics cart 24 in which a photo or other copy of the optically readable label is scanned for an unauthorized tool or endoscopic image capture device 500. By having the optical scanner in the socket 27, spoofing the electronics cart 24 is made harder due to the difficulty in providing both the copy of the optically readable label and the connector 502 to the socket 27 at the same time.

In a further implementation, the electronics cart 24 comprises the optical scanner at a location other than in the socket 27 or other mating receptacle. For example, the optical scanner may be located on a surface of the electronics cart 24 adjacent to the socket 27 or in a hand-held scanner device coupled to the electronics cart 24. The optical scanner may also be located on other devices than the electronics cart 24, such as the manipulating system 54 and/or the computer processor 58 as noted above. By positioning the optical scanner at a location outside of the socket 27, a corresponding set of calibration data for an endoscopic image capture device 500 or other tool can be retrieved prior to use or insertion into the socket 27. In this way, the corresponding set of calibration data is retrieved without counting as a use of the endoscopic image capture device 500 or other tool upon the optical scanner scanning the optically readable label. Also, additional endoscopic image capture devices 500 or other tools may be scanned to retrieve corresponding sets of calibration data while an endoscopic image capture device 500 or other tool is in use or otherwise already inserted into the socket 27.

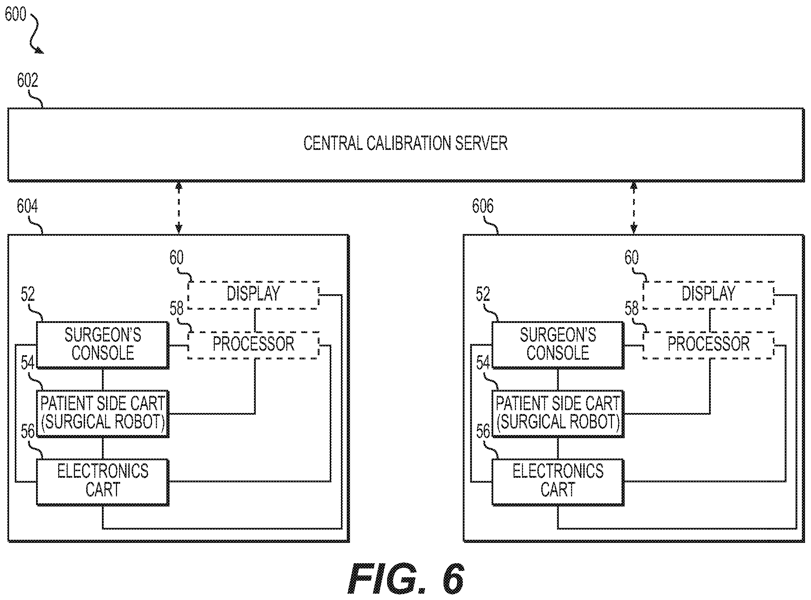

FIG. 6 is a diagrammatic illustration of an image capture device calibration tool 600. The image capture device calibration tool 600 includes a first teleoperated surgical system 604 and a second teleoperated surgical system 606 in data communication with a central calibration server 602. Teleoperated surgical systems 604, 606 are similar to teleoperated surgical system 50 shown at FIG. 4 and described above. In some implementations, the teleoperated surgical systems 604, 606 have the same system configuration. In some implementations, the teleoperated surgical systems 604, 606 have different system configurations. For example, the teleoperated surgical systems 604, 606 may have a difference in one or more of an image processor, vision system, or other hardware system, a software release version, and/or a light source with a different spectral response. Other differences that impact the calibration of an image capture device are contemplated by this disclosure.

While only two teleoperated surgical systems 604, 606 are shown in FIG. 6, more or fewer systems may be in data communication with the central calibration server 602. In some implementations, the teleoperated surgical systems 604, 606 may be located at the same or different health care provider locations. For example, the first teleoperated surgical system 604 may be located at a first health care provider location and the second teleoperated surgical system 606 may be located at a second health care provider location.

The central calibration server 602 comprises or is in communication with one or more databases that store calibration records. Each calibration record is indexed against a unique identifier of a corresponding image capture device. A calibration record includes one or more sets of calibration data and a set of test pattern images. Different sets of calibration data may be optimized for different procedures or controller system configurations. Each set of calibration data comprises calibration parameters for adjusting captured images based on one or more of an optical distortion, color mapping correction, a color of light illuminating a scene, and/or stereoscopic image alignment. Other calibration parameters may be used. For example, calibration parameters may include image sensor characteristics, such as dead or stuck pixels, a cropping region, analog gains, digital gains, or other such image sensor characteristics.

In some implementations, different sets of calibration parameters may increase performance in areas of interest during a particular procedure, while potentially reducing performance in other areas of functionality. For example, if a particular procedure has a need for a very fine close up image, the calibration parameters for the set of calibration data for the procedure may be optimized for sharpness of images processed using the calibration data. Such optimization for the sharpness of images may come at the expense of color accuracy or fidelity. Similarly, the functionality of a lower-capability image capture device may be extended through a set of calibration data that increases performance of the lower-capability image capture device in a certain area. The use of sets of calibration data with particular performance enhancements may be based on requirements for a particular procedure, preferences of an end-user (e.g., surgeon), or any other basis.

The set of test pattern images includes images captured by the corresponding image capture device in a calibration environment, such as at a factory calibration of the corresponding image capture device. Each image in the set of test pattern images may capture a different test pattern and/or a different lighting condition. In some implementations, a subset of the test pattern images may be captured for calibrating the image capture device for a particular procedure, a particular lighting condition, or a particular controller system configuration.

The central calibration server 602 also comprises or is in communication with a calibration processor. The calibration processor is configured to generate a set of calibration data from one or more images in the set of test pattern images. The calibration processor may dynamically generate a set of calibration data in response to a request for calibration data from a controller system, such as a request from the electronics cart 56 or the processor 58 in one of the teleoperated surgical systems 604, 606.

Alternatively or additionally, the calibration processor may generate a set of calibration data in response to a change in a calibration condition. For example, the change in a calibration condition may be based on a change in a system configuration of one of the teleoperated surgical systems 604, 606 or a change in a transform used to generate the calibration data.

A change in the system configuration may include a change in one or more of an image processor, vision system, or other hardware component, a software release version, and/or a light source in a controller system. The change in the system configuration may be caused from an upgrade or maintenance on one of the teleoperated surgical systems 604, 606. The change in the system configuration may also be caused by connecting an image capture device to a different controller system.

For example, an image capture device may initially be connected to a first electronics cart 56 with a first system configuration and a first set of calibration data is provided for the first system configuration. At a later time, the image capture device may be connected to a second electronics cart 56 with a second system configuration that is different than the first system configuration. The second system configuration may have a different image processor, vision system, or other hardware component, a different software release version, and/or a different light source than the first system configuration. Accordingly, the calibration processor may generate a second set of calibration data for the image capture device that is optimized for the second system configuration.

The change in the system configuration of a controller system may be identified by the calibration processor as part of processing the request for calibration data from the controller system. For example, the request from the controller system may include information about a current system configuration of the controller system. Alternatively, upon implementation of a system configuration change of the controller system, a notification may be sent from the controller system to the central calibration server 602.

Other calibration events may also trigger the calibration processor to generate a set of calibration data. For example, a calibration event may be received by the central calibration server 602 upon a new image capture device being ordered, delivered, or received at a health care provider location of one of the teleoperated surgical systems 604, 606. For example, upon a new image capture device being ordered, delivered, or received at the first health care provider location of the teleoperated surgical system 604, the central calibration server receives a calibration notification of the new image capture device. The calibration processor generates a set of calibration data for the new image capture device for use with the electronics cart 56 and/or processor 58. The central calibration server 602 may deliver or otherwise push the newly generated set of calibration data to the electronics cart 56 and/or processor 58 in the first teleoperated surgical system 604.

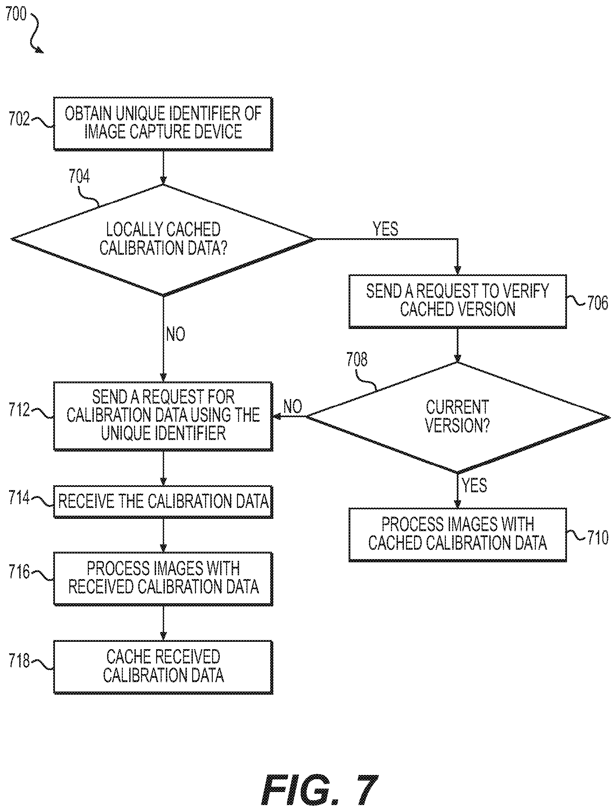

FIG. 7 is a flowchart illustrating an example process 700 of obtaining calibration data for an image capture device. The process 700 is executed by the controller system, such as the electronics cart 56 and/or processor 58. Other components or systems of a teleoperated surgical system may execute the process 700. In the interest of simplicity, the process 700 is described below as being executed by the electronics cart 56, but is not limiting to the sprit or scope of the disclosure.

At 702, the electronics cart 56 obtains a unique identifier of an image capture device. For example, upon the connector 502 of the endoscopic image capture device 500 being inserted into the socket 27 of the electronics cart 56, the electronics cart 56 obtains the unique identifier of the endoscopic image capture device 500. As discussed above, the unique identifier may be obtained from reading a memory, a wireless tag, or an optical label by the electronics cart 56 or an external reader in communication with the electronics cart 56.

At 704, the electronics cart 56 checks a local calibration cache against the obtained unique identifier for calibration data for the image capture device. At 706, if a local copy of the calibration data for the image capture device is maintained in the cache, the electronics cart 56 sends a request to the central calibration server 602 to verify that the cached version of the calibration data is current.

For example, the electronics cart 56 may request a hash of a current version of the calibration data from the central calibration server 602. The electronics cart 56 may also determine or read a hash of the local copy of the calibration data for comparison against the hash received from the central calibration server 602. Alternatively, the electronics cart 56 may send a hash of the local copy of the calibration data to the central calibration server 602 and receive a result of a comparison performed by the central calibration server 602.

At 708, the electronics cart 56 determines whether the local copy of the calibration data is current. For example, based on a result of the comparison of the hash of the local copy against the hash received from the central calibration server 602 or based on the received result of the comparison performed by the central calibration server 602.

At 710, if the electronics cart 56 determines that the local copy of the calibration data is current, then the local copy of the calibration data is used to process images received from the image capture device.

Otherwise, at 712, the electronics cart 56 sends a request to the central calibration server 602 for calibration data based on the obtained unique identifier of the image capture device. Likewise, if at 704, it is determined that a local copy of the calibration data is not present in the cache, the electronics cart 56 sends a request to the central calibration server 602 for calibration data based on the obtained unique identifier of the image capture device.

At 714, the electronics cart 56 receives the requested calibration data. At 716, the electronics cart 56 uses the received calibration data to process images received from the image capture device. At 718, the received calibration data is stored in the local calibration cache and indexed against the unique identifier of the image capture device.

One or more of the features of process 700 may be optional or performed in a different order. For example, if the electronics cart 56 does not have a local calibration cache, then the features performed at 704-710 may be omitted. As another example, the feature performed at 718 may be performed prior to the feature performed at 716. Other additions or variations of the process 700 are contemplated by this disclosure.

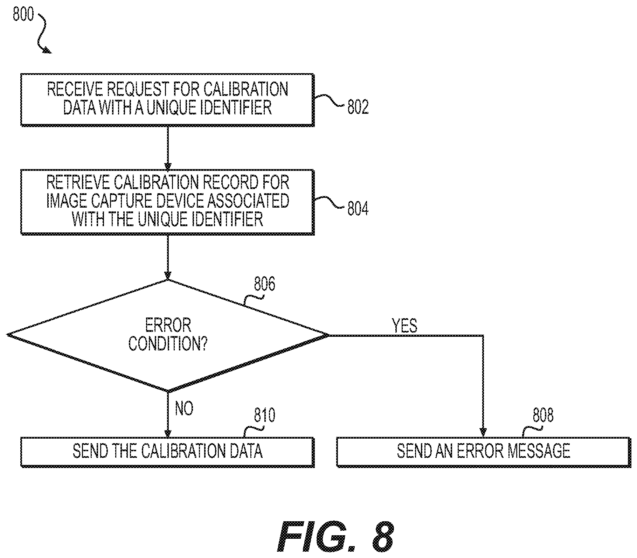

FIG. 8 is a flowchart illustrating an example process 800 of sending calibration data in response to a request. The process 800 is executed by the central calibration server 602 and/or one or more processors in communication with the central calibration server 602. In the interest of simplicity, the process 800 is described below as being executed by the central calibration server 602, but is not limiting to the sprit or scope of the disclosure.

At 802, the central calibration server 602 receives a request for calibration data from a controller system, such as the electronics cart 56 and/or processor 58. The request includes a unique identifier of an image capture device. The request may additionally include information about the controller system, a configuration of the controller system, or a procedure to be performed with the controller system.

At 804, using the received unique identifier, the central calibration server 602 retrieves a set of calibration data for the image capture device from a database on or in communication with the central calibration server 602. For example, the central calibration server 602 performs a look-up operation for one or more tables of calibration data indexed against the unique identifier. Additional data received from the request or otherwise obtained by the central calibration server 602 may be used to retrieve a particular set of calibration data from among a plurality of sets of calibration data. For example, upon receiving an indication that the image capture device will be used for a particular procedure, the central calibration server 602 may retrieve a set of calibration data optimized for the performance of the particular procedure.

At 806, the central calibration server 602 determines if any error conditions exist associated with the image capture device. For example, the central calibration server 602 may enforce policies to prevent use of the controller system with non-registered image capture devices, limit a number of uses of the image capture device before maintenance or refurbishment is required, or lock out image capture devices subject to a recall.

At 808, if an error condition exists, the central calibration server 602 replies to the received request for calibration data with an error message. For example, the error message may indicate that the image capture device is incompatible with the controller system, that maintenance or refurbishment of the image capture device is required, or that the image capture device is subject to a recall. Other error conditions and error messages are contemplated by this disclosure.

At 810, if the central calibration server 602 determines that no error condition exists for the image capture device, a reply is sent to the controller system with the retrieved set of calibration data.

One or more of the features of process 800 may be optional or performed in a different order. Other additions or variations of the process 800 are contemplated by this disclosure.

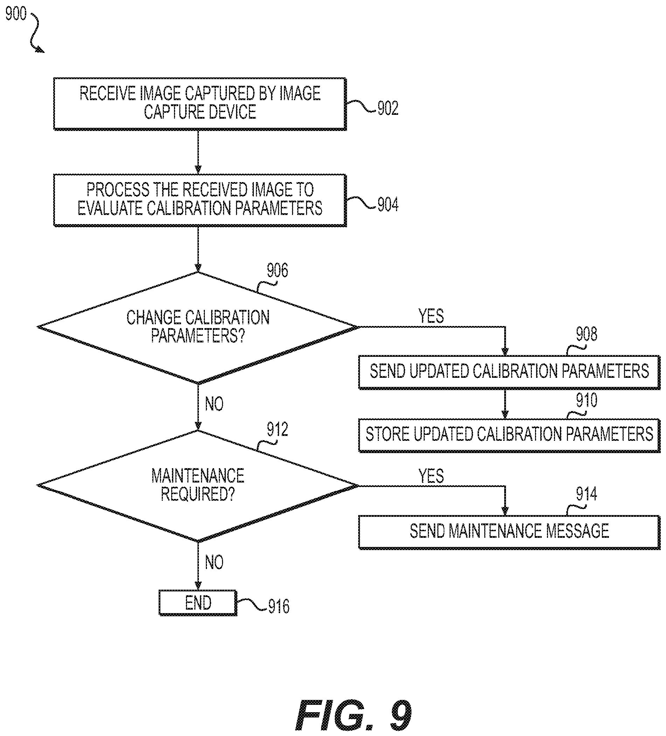

FIG. 9 is a flowchart illustrating an example process 900 of determining changed calibration parameters or maintenance conditions based on field images captured by an image capture device. In use, a controller system, such as the electronics cart 56 and/or processor 58, may periodically provide field images captured by an image capture device during a procedure to the central calibration server 602. For example, the controller system may provide at least one image captured by the image capture device for each lighting condition used during a procedure. Other numbers of images and frequency of providing images to the central calibration server 602 may be used.

At 902, the central calibration server 602 receives a field image from a controller system. The field image may be received along with a unique identifier of an image capture device that captured the field image. Additional information regarding the configuration of the controller system, a current lighting condition, or other context information for the conditions in which the field image was captured may also be provided.

At 904, the central calibration server 602 processes the received field image to evaluate one or more calibration parameters for the image capture device. For example, a plurality of received field images may be processed to identify changes over time. For example, processing of the field images may reveal spectral attenuation in the captured images over time due to wear of the fiber optic bundle in the flexible cable 506. Likewise, processing of the field images may reveal shifts in the color space over time of one or more image sensors in the image capture device. Additionally, processing of the field images may reveal alignment issues in stereoscopic images.

At 906, the central calibration server 602 determines if one or more calibration parameters of the calibration data should be changed based on processing the field images. If so, at 908, the central calibration server 602 determines and sends updated calibration parameters to the controller system. For example, the updated calibration parameters may adjust a brightness, a color space, an alignment or other features of images process by the updated calibration data. The central calibration server 602 may also send one or more updated operational parameters for the controller system or image capture device. For example, in response to detecting spectral attenuation in the field images, the central calibration server may send operational parameters to increase a power of a light source provided by the controller system or to increase a gain of an image sensor of the image capture device. Other variations of operational parameters may be used.

At 910, the central calibration server 602 stores the updated calibration parameters of the calibration data in the calibration record associated with the image capture device. For example, calibration records associated with the unique identifier on one or more databases maintained by or in communication with the central calibration server 602 are updated.

If the central calibration server 602 determines that no calibration parameters are to be changed at 906, the central calibration server 602 determines whether any maintenance conditions exist at 912. For example, upon detecting a stereoscopic alignment issue in the processed field images, the central calibration server may determine that the image capture device requires maintenance. Other maintenance conditions are contemplated by this disclosure.

At 914, the central calibration server 602 sends a maintenance message to the controller system. For example, the maintenance message may provide instructions for adjusting one or more operational parameters, sending the image capture device for maintenance or refurbishment, or otherwise providing a notification of required maintenance. Otherwise, if the central calibration server 602 determines that no maintenance conditions exist, the process 900 ends at 916.

One or more of the features of process 900 may be optional or performed in a different order. Other additions or variations of the process 900 are contemplated by this disclosure.

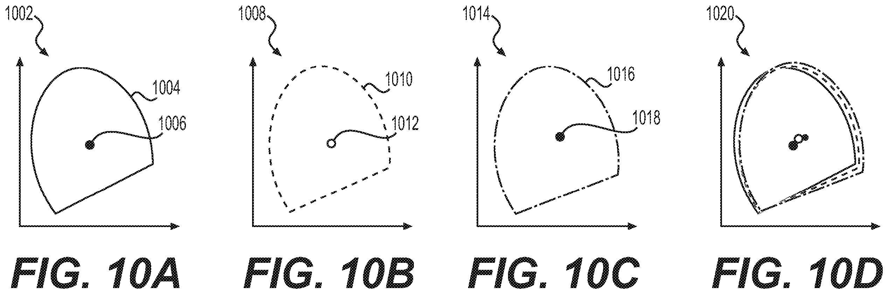

FIGS. 10A-10D are chromaticity diagrams generated from processed field images showing a shift in a center of the color space of an image capture device. Each of the chromaticity diagrams may be generated from a set of field images received and processed by the central calibration server 602.

For example, as shown in FIG. 10A, a first chromaticity diagram 1002 is generated by the central calibration server 602 upon processing a first set of received field images of an image capture device. The chromaticity diagram 1002 shows a first color space 1004 and a center of the first color space 1006 determined from the first set of received field images. FIG. 10B shows a second chromaticity diagram 1008 is generated by the central calibration server 602 upon processing a second set of received field images of the image capture device. The chromaticity diagram 1008 shows a second color space 1010 and a center of the second color space 1012 determined from the second set of received field images. FIG. 10C shows a third chromaticity diagram 1014 is generated by the central calibration server 602 upon processing a third set of received field images of the image capture device. The chromaticity diagram 1014 shows a third color space 1016 and a center of the third color space 1018 determined from the third set of received field images. The first, second, and third set of received filed images may be received at different times.

FIG. 10D shows a chromaticity diagram 1020 with the first, second, and third color spaces 1004, 1010, 1016 overlaid upon each other and showing a track of the center of the first, second, and third color spaces 1006, 1012, 1018. As shown in FIG. 10D, the center of the of the first, second, and third color spaces 1006, 1012, 1018 shows a shift in the color space of the image capture device over time. As discussed above, updated calibration parameters may be generated by the central calibration server 602 based on this detected shift in the color space of the image capture device.

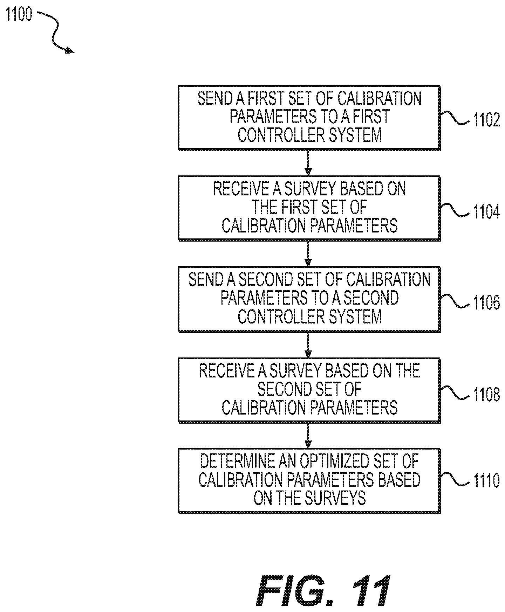

FIG. 11 is a flowchart illustrating an example process 1100 of conducting a calibration parameter survey. At 1102, the central calibration server 602 sends a first set of calibration parameters to a first controller system, such as the electronics cart 56 and/or processor 58 of the first teleoperated surgical system 604. At 1104, the central calibration server 602 receives one or more survey results from the first controller system based on user feedback provided in response to the first set of calibration parameters.

At 1106, the central calibration server 602 sends a second set of calibration parameters to a second controller system, such as the electronics cart 56 and/or processor 58 of the second teleoperated surgical system 606. At 1108, the central calibration server 602 receives one or more survey results from the second controller system based on user feedback provided in response to the second set of calibration parameters.

At 1110, the central calibration server 602 processes the surveys received in response to the first and second set of calibration parameters and determines an optimized set of calibration parameters based on the received surveys. For example, the central calibration server 602 may select from highest rated calibration parameters from among the first and second set of calibration parameters to determine the optimized set of calibration parameters. Other methods of processing the survey results to determine the optimized set of calibration parameters may be used.

One or more of the features of process 1100 may be optional or performed in a different order. For example, the features performed at 1106-1 108 may be performed prior to the features performed at 1102-1104. Other additions or variations of the process 1100 are contemplated by this disclosure.

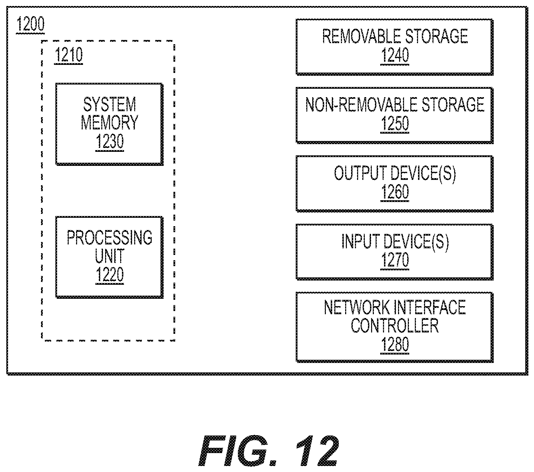

It should be appreciated that the logical operations described herein with respect to the various figures may be implemented (1) as a sequence of computer implemented acts or program modules (i.e., software) running on a computing device (e.g., the computing device described in FIG. 12), (2) as interconnected machine logic circuits or circuit modules (i.e., hardware) within the computing device and/or (3) a combination of software and hardware of the computing device. Thus, the logical operations discussed herein are not limited to any specific combination of hardware and software. The implementation is a matter of choice dependent on the performance and other requirements of the computing device. Accordingly, the logical operations described herein are referred to variously as operations, structural devices, acts, or modules. These operations, structural devices, acts and modules may be implemented in software, in firmware, in special purpose digital logic, and any combination thereof. It should also be appreciated that more or fewer operations may be performed than shown in the figures and described herein. These operations may also be performed in a different order than those described herein.

Referring to FIG. 12, an example computing device 1200 upon which embodiments of the invention may be implemented is illustrated. For example, each of the computer processor located on an electronics cart 56 or electronics car 24, computer processor 58, and central calibration server 602 described herein may each be implemented as a computing device, such as computing device 1200. It should be understood that the example computing device 1200 is only one example of a suitable computing environment upon which embodiments of the invention may be implemented. Optionally, the computing device 1200 can be a well-known computing system including, but not limited to, personal computers, servers, handheld or laptop devices, multiprocessor systems, microprocessor-based systems, network personal computers (PCs), minicomputers, mainframe computers, embedded systems, and/or distributed computing environments including a plurality of any of the above systems or devices. Distributed computing environments enable remote computing devices, which are connected to a communication network or other data transmission medium, to perform various tasks. In the distributed computing environment, the program modules, applications, and other data may be stored on local and/or remote computer storage media.

In an embodiment, the computing device 1200 may comprise two or more computers in communication with each other that collaborate to perform a task. For example, but not by way of limitation, an application may be partitioned in such a way as to permit concurrent and/or parallel processing of the instructions of the application. Alternatively, the data processed by the application may be partitioned in such a way as to permit concurrent and/or parallel processing of different portions of a data set by the two or more computers. In an embodiment, virtualization software may be employed by the computing device 1200 to provide the functionality of a number of servers that is not directly bound to the number of computers in the computing device 1200. For example, virtualization software may provide twenty virtual servers on four physical computers. In an embodiment, the functionality disclosed above may be provided by executing the application and/or applications in a cloud computing environment. Cloud computing may comprise providing computing services via a network connection using dynamically scalable computing resources. Cloud computing may be supported, at least in part, by virtualization software. A cloud computing environment may be established by an enterprise and/or may be hired on an as-needed basis from a third-party provider. Some cloud computing environments may comprise cloud computing resources owned and operated by the enterprise as well as cloud computing resources hired and/or leased from a third-party provider.

In its most basic configuration, computing device 1200 typically includes at least one processing unit 1220 and system memory 1230. Depending on the exact configuration and type of computing device, system memory 1230 may be volatile (such as random-access memory (RAM)), non-volatile (such as read-only memory (ROM), flash memory, etc.), or some combination of the two. This most basic configuration is illustrated in FIG. 12 by dashed line 1210. The processing unit 1220 may be a standard programmable processor that performs arithmetic and logic operations necessary for operation of the computing device 1200. While only one processing unit 1220 is shown, multiple processors may be present. Thus, while instructions may be discussed as executed by a processor, the instructions may be executed simultaneously, serially, or otherwise executed by one or multiple processors. The computing device 1200 may also include a bus or other communication mechanism for communicating information among various components of the computing device 1200.

Computing device 1200 may have additional features/functionality. For example, computing device 1200 may include additional storage such as removable storage 1240 and non-removable storage 1250 including, but not limited to, magnetic or optical disks or tapes. Computing device 1200 may also contain network connection(s) 1280 that allow the device to communicate with other devices such as over the communication pathways described herein. The network connection(s) 1280 may take the form of modems, modem banks, Ethernet cards, universal serial bus (USB) interface cards, serial interfaces, token ring cards, fiber distributed data interface (FDDI) cards, wireless local area network (WLAN) cards, radio transceiver cards such as code division multiple access (CDMA), global system for mobile communications (GSM), long-term evolution (LTE), worldwide interoperability for microwave access (WiMAX), and/or other air interface protocol radio transceiver cards, and other well-known network devices. Computing device 1200 may also have input device(s) 1270 such as a keyboards, keypads, switches, dials, mice, track balls, touch screens, voice recognizers, card readers, paper tape readers, or other well-known input devices. Output device(s) 1260 such as a printers, video monitors, liquid crystal displays (LCDs), touch screen displays, displays, speakers, etc. may also be included. The additional devices may be connected to the bus in order to facilitate communication of data among the components of the computing device 1200. All these devices are well known in the art and need not be discussed at length here.

The processing unit 1220 may be configured to execute program code encoded in tangible, computer-readable media. Tangible, computer-readable media refers to any media that is capable of providing data that causes the computing device 1200 (i.e., a machine) to operate in a particular fashion. Various computer-readable media may be utilized to provide instructions to the processing unit 1220 for execution. Example tangible, computer-readable media may include, but is not limited to, volatile media, non-volatile media, removable media and non-removable media implemented in any method or technology for storage of information such as computer readable instructions, data structures, program modules or other data. System memory 1230, removable storage 1240, and non-removable storage 1250 are all examples of tangible, computer storage media. Example tangible, computer-readable recording media include, but are not limited to, an integrated circuit (e.g., field-programmable gate array or application-specific IC), a hard disk, an optical disk, a magneto-optical disk, a floppy disk, a magnetic tape, a holographic storage medium, a solid-state device, RAM, ROM, electrically erasable program read-only memory (EEPROM), flash memory or other memory technology, CD-ROM, digital versatile disks (DVD) or other optical storage, magnetic cassettes, magnetic tape, magnetic disk storage or other magnetic storage devices.

It is fundamental to the electrical engineering and software engineering arts that functionality that can be implemented by loading executable software into a computer can be converted to a hardware implementation by well-known design rules. Decisions between implementing a concept in software versus hardware typically hinge on considerations of stability of the design and numbers of units to be produced rather than any issues involved in translating from the software domain to the hardware domain. Generally, a design that is still subject to frequent change may be preferred to be implemented in software, because re-spinning a hardware implementation is more expensive than re-spinning a software design. Generally, a design that is stable that will be produced in large volume may be preferred to be implemented in hardware, for example in an application specific integrated circuit (ASIC), because for large production runs the hardware implementation may be less expensive than the software implementation. Often a design may be developed and tested in a software form and later transformed, by well-known design rules, to an equivalent hardware implementation in an application specific integrated circuit that hardwires the instructions of the software. In the same manner as a machine controlled by a new ASIC is a particular machine or apparatus, likewise a computer that has been programmed and/or loaded with executable instructions may be viewed as a particular machine or apparatus.

In an example implementation, the processing unit 1220 may execute program code stored in the system memory 1230. For example, the bus may carry data to the system memory 1230, from which the processing unit 1220 receives and executes instructions. The data received by the system memory 1230 may optionally be stored on the removable storage 1240 or the non-removable storage 1250 before or after execution by the processing unit 1220.

It should be understood that the various techniques described herein may be implemented in connection with hardware or software or, where appropriate, with a combination thereof. Thus, the methods and apparatuses of the presently disclosed subject matter, or certain aspects or portions thereof, may take the form of program code (i.e., instructions) embodied in tangible media, such as floppy diskettes, CD-ROMs, hard drives, or any other machine-readable storage medium wherein, when the program code is loaded into and executed by a machine, such as a computing device, the machine becomes an apparatus for practicing the presently disclosed subject matter. In the case of program code execution on programmable computers, the computing device generally includes a processor, a storage medium readable by the processor (including volatile and non-volatile memory and/or storage elements), at least one input device, and at least one output device. One or more programs may implement or utilize the processes described in connection with the presently disclosed subject matter, e.g., through the use of an application programming interface (API), reusable controls, or the like. Such programs may be implemented in a high level procedural or object-oriented programming language to communicate with a computer system. However, the program(s) can be implemented in assembly or machine language, if desired. In any case, the language may be a compiled or interpreted language and it may be combined with hardware implementations.

Embodiments of the methods and systems may be described herein with reference to block diagrams and flowchart illustrations of methods, systems, apparatuses and computer program products. It will be understood that each block of the block diagrams and flowchart illustrations, and combinations of blocks in the block diagrams and flowchart illustrations, respectively, can be implemented by computer program instructions. These computer program instructions may be loaded onto a general-purpose computer, special purpose computer, or other programmable data processing apparatus to produce a machine, such that the instructions which execute on the computer or other programmable data processing apparatus create a means for implementing the functions specified in the flowchart block or blocks.

These computer program instructions may also be stored in a computer-readable memory that can direct a computer or other programmable data processing apparatus to function in a particular manner, such that the instructions stored in the computer-readable memory produce an article of manufacture including computer-readable instructions for implementing the function specified in the flowchart block or blocks. The computer program instructions may also be loaded onto a computer or other programmable data processing apparatus to cause a series of operational steps to be performed on the computer or other programmable apparatus to produce a computer-implemented process such that the instructions that execute on the computer or other programmable apparatus provide steps for implementing the functions specified in the flowchart block or blocks.

Accordingly, blocks of the block diagrams and flowchart illustrations support combinations of means for performing the specified functions, combinations of steps for performing the specified functions and program instruction means for performing the specified functions. It will also be understood that each block of the block diagrams and flowchart illustrations, and combinations of blocks in the block diagrams and flowchart illustrations, can be implemented by special purpose hardware-based computer systems that perform the specified functions or steps, or combinations of special purpose hardware and computer instructions.

While several embodiments have been provided in the present disclosure, it should be understood that the disclosed systems and methods may be embodied in many other specific forms without departing from the spirit or scope of the present disclosure. The present examples are to be considered as illustrative and not restrictive, and the intention is not to be limited to the details given herein. For example, the various elements or components may be combined or integrated in another system or certain features may be omitted or not implemented.

Also, techniques, systems, subsystems, and methods described and illustrated in the various embodiments as discrete or separate may be combined or integrated with other systems, modules, techniques, or methods without departing from the scope of the present disclosure. Other items shown or discussed as directly coupled or communicating with each other may be indirectly coupled or communicating through some interface, device, or intermediate component, whether electrically, mechanically, or otherwise. Other examples of changes, substitutions, and alterations are ascertainable by one skilled in the art and could be made without departing from the spirit and scope disclosed herein. The present invention provides a system and method as set out in the independent claims. Advantageous embodiments are defined in the dependent claims.

## Claims

1. A system, comprising:
a network accessible database (602) configured to store calibration data for an image capture device (500);
a controller system (24) comprising an image processor configured to receive images captured by the image capture device, the image processor further configured to retrieve the calibration data from the network accessible database via a network connection, and the image processor further configured to process the received images based on the retrieved calibration data; and
a calibration processor in communication with the network accessible database (602), the calibration processor configured to receive calibration images captured by the image capture device (500) and generate the calibration data responsive to a change in a configuration of the controller system (24), wherein the calibration data is generated based on applying a calibration algorithm to one or more of the calibration images, wherein the calibration algorithm is selected based on one or more of: a type of procedure in which the image capture device (500) is to be used, the configuration of the controller system (24), or availability of a different calibration methodology for calculating one or more parameters of the calibration data.

2. The system of claim 1, further comprising:
a tool comprising the image capture device (500) and electrically coupled to the controller system (24), wherein the tool has a unique identifier;
preferably, wherein the calibration data is indexed with the image capture device in the network accessible database based on the unique identifier;
more preferably, wherein the unique identifier is maintained by the tool, and wherein the image processor is further configured to obtain the unique identifier from the tool.

3. The system of claim 2, wherein the unique identifier is maintained by the tool on a memory device of the tool, on a wireless tag of the tool, or on a visual indicator on a surface of the tool.

4. The system of claim 3, wherein the image processor further comprises an optical scanner configured to read the visual indicator from the surface of the tool to obtain the unique identifier from the tool.

5. The system of claim 4, wherein the optical scanner is located in a mating receptacle configured to couple the tool to the controller system;
preferably, wherein the optical scanner is a camera or a bar code reader.

6. The system of any of claims 3-5, wherein the visual indicator is printed, adhered, labeled, embossed, or stamped onto the surface of the tool;
optionally, wherein the visual indicator is an alphanumeric serial number, a linear barcode that encodes the unique identifier, a two-dimensional barcode that encodes the unique identifier, or image encoded with the unique identifier.

7. The system of any of claims 2-5, wherein the tool comprises an endoscope (28), and the image capture device comprises a pair of image sensors configured to capture stereoscopic image data of a surgical site during a surgical procedure.

8. The system of any of claims 1-5, wherein the calibration data comprises data for processing the received images to correct the received images based on one or more of a color of light emitted by an illuminator configured to illuminate a scene captured by the received images, calibration images captured by the image capture device, optical distortions introduced by an optical system of the image capture device, a stereoscopic image calibration, a color space mapping, or an image sensor characteristic of the image capture device.

9. The system of claim 1, wherein the calibration images are stored in the network accessible database (602) with the calibration data,
optionally, wherein the calibration data is different from a prior set of calibration data for the image capture device, wherein the prior set of calibration data is generated by the calibration processor based on applying a different calibration algorithm to one or more of the calibration images,
optionally, wherein the calibration processor is further configured to receive a user feedback survey evaluating the calibration data in comparison to the prior set of calibration data.

10. The system of claim 1, wherein the configuration of the controller system comprises one or more of an illumination source provided by the controller system, a type of the image processor, or a software release version of the controller system.

11. The system of claim 10, wherein the calibration data is generated to enhance an image feature significant to the type of procedure in which the image capture device is to be used,
optionally, wherein the image feature is one or more of a focus level, sharpness, color fidelity, or color accuracy.

12. The system of claim 10, wherein the calibration processor is further configured to retrieve an operational image captured by the image capture device (500) during the procedure from the controller system and to adjust the calibration data based on the operational images,
optionally, wherein a luminance value of the operational image is compared to an expected luminance value determined based on a known distance to an object captured in the operational image and a known power output to an illumination source that is illuminating the object captured in the operational image,
optionally, wherein a color space of the operation image is compared to an expected color space of the image capture device to determine whether a center of the color space is offset from a center of the expected color space,
optionally, wherein a stereoscopic alignment of the operational image is evaluated to identify misalignment with a corresponding operational image.

13. The system of any of claims 1-5, wherein the controller system further comprises a calibration cache memory configured to store the calibration data retrieved from the network accessible database (602),
optionally, wherein the controller system is further configured to store calibration data of image capture devices known to have been shipped to a location associated with a location of the controller system,
optionally, wherein the controller system is further configured to store calibration data of a predetermined number of previously used image capture devices (500),
optionally, wherein the controller system is further configured to store default calibration data for a type of the image capture device (500),
optionally, wherein the controller system is further configured to compare a cached set of calibration data for the image capture device to the calibration data stored on the network accessible database prior to retrieving the calibration data from the network accessible database.

14. A method, comprising:
receiving, by a calibration processor in communication with a network accessible database (602), from a controller system (24) for an image capture device (500), calibration images captured by the image capture device (500);
generating, by the calibration processor, calibration data responsive to a change in a configuration of the controller system (24), wherein the calibration data is generated based on applying a calibration algorithm to one or more of the calibration images, wherein the calibration algorithm is selected based on one or more of: a type of procedure in which the image capture device (500) is to be used, the configuration of the controller system (24), or availability of a different calibration methodology for calculating one or more parameters of the calibration data;
storing the calibration data for the image capture device (500) in the network accessible database (602);
retrieving, by the controller system (24), the calibration data from the network accessible database via a network connection;
receiving, by the controller system, second images captured by the image capture device (500); and
processing, by an image processor of the controller system (24), the received second images based on the retrieved calibration data.

## Patentansprüche

1. System, das Folgendes umfasst:
eine über ein Netzwerk zugängliche Datenbank (602), die zum Speichern von Kalibrierungsdaten für ein Bildaufnahmegerät (500) konfiguriert ist;
ein Steuersystem (24), das einen Bildprozessor umfasst, der zum Empfangen von von dem Bildaufnahmegerät aufgenommenen Bildern konfiguriert ist, wobei der Bildprozessor ferner zum Abrufen der Kalibrierungsdaten aus der über ein Netzwerk zugänglichen Datenbank über eine Netzwerkverbindung konfiguriert ist und der Bildprozessor ferner zum Verarbeiten der empfangenen Bilder auf der Basis der abgerufenen Kalibrierungsdaten konfiguriert ist; und
einen Kalibrierungsprozessor in Kommunikation mit der über das Netzwerk zugänglichen Datenbank (602), wobei der Kalibrierungsprozessor zum Empfangen von von dem Bildaufnahmegerät (500) aufgenommenen Kalibrierungsbildern und zum Erzeugen der Kalibrierungsdaten als Reaktion auf eine Änderung in einer Konfiguration des Steuersystems (24) konfiguriert ist, wobei die Kalibrierungsdaten auf der Basis der Anwendung eines Kalibrierungsalgorithmus auf eines oder mehrere der Kalibrierungsbilder erzeugt werden, wobei der Kalibrierungsalgorithmus ausgewählt wird auf der Basis von einem oder mehreren von einem Typ von Prozedur, in dem das Bildaufnahmegerät (500) verwendet werden soll, der Konfiguration des Steuersystems (24) und der Verfügbarkeit einer anderen Kalibrierungsmethodik zum Berechnen eines oder mehrerer Parameter der Kalibrierungsdaten.

2. System nach Anspruch 1, das ferner Folgendes umfasst:
ein Werkzeug, das das Bildaufnahmegerät (500) umfasst und elektrisch mit dem Steuersystem (24) gekoppelt ist, wobei das Werkzeug eine eindeutige Kennung aufweist;
wobei die Kalibrierungsdaten vorzugsweise auf der Basis der eindeutigen Kennung mit dem Bildaufnahmegerät in der über das Netzwerk zugänglichen Datenbank indiziert sind;
wobei stärker bevorzugt die eindeutige Kennung von dem Werkzeug geführt wird, und wobei der Bildprozessor ferner zum Erhalten der eindeutigen Kennung von dem Werkzeug konfiguriert ist.

3. System nach Anspruch 2, wobei die eindeutige Kennung von dem Werkzeug auf einem Speichergerät des Werkzeugs, auf einem drahtlosen Tag des Werkzeugs oder auf einer visuellen Anzeige auf einer Oberfläche des Werkzeugs geführt wird.

4. System nach Anspruch 3, wobei der Bildprozessor ferner einen optischen Scanner umfasst, der zum Lesen der visuellen Anzeige von der Oberfläche des Werkzeugs zum Erhalten der eindeutigen Kennung von dem Werkzeug konfiguriert ist.

5. System nach Anspruch 4, wobei sich der optische Scanner in einer passenden Aufnahme befindet, die zum Koppeln des Werkzeugs mit dem Steuersystem konfiguriert ist;
wobei der optische Scanner vorzugsweise eine Kamera oder ein Barcodeleser ist.

6. System nach einem der Ansprüche 3-5, wobei die visuelle Anzeige auf die Oberfläche des Werkzeugs gedruckt, geklebt, etikettiert, geprägt oder gestempelt ist;
wobei die visuelle Anzeige optional eine alphanumerische Seriennummer, ein die eindeutige Kennung codierender linearer Barcode, ein die eindeutige Kennung codierender zweidimensionaler Barcode oder ein mit der eindeutigen Kennung codiertes Bild ist.

7. System nach einem der Ansprüche 2-5, wobei das Werkzeug ein Endoskop (28) umfasst und das Bildaufnahmegerät ein Paar Bildsensoren umfasst, die zum Erfassen stereoskopischer Bilddaten einer Operationsstelle während einer chirurgischen Prozedur konfiguriert sind.

8. System nach einem der Ansprüche 1-5, wobei die Kalibrierungsdaten Daten zum Verarbeiten der empfangenen Bilder zum Korrigieren der empfangenen Bilder auf der Basis von einem oder mehreren von einer Farbe von Licht, das von einem Illuminator emittiert wird, der zum Beleuchten eines von den empfangenen Bildern erfassten Szene konfiguriert ist, von dem Bildaufnahmegerät aufgenommenen Kalibrierungsbildern, durch ein optisches System des Bildaufnahmegeräts verursachten optischen Verzerrungen, einer stereoskopischen Bildkalibrierung, einer Farbraumzuordnung oder einer Bildsensorcharakteristik des Bildaufnahmegeräts umfassen.

9. System nach Anspruch 1, wobei die Kalibrierungsbilder mit den Kalibrierungsdaten in der über das Netzwerk zugänglichen Datenbank (602) gespeichert sind,
wobei sich optional die Kalibrierungsdaten von einem vorherigen Satz von Kalibrierungsdaten für das Bildaufnahmegerät unterscheiden, wobei der vorherige Satz von Kalibrierungsdaten durch den Kalibrierungsprozessor auf der Basis der Anwendung eines anderen Kalibrierungsalgorithmus auf eines oder mehrere der Kalibrierungsbilder erzeugt wird,
wobei der Kalibrierungsprozessor optional ferner zum Empfangen einer Benutzer-Feedback-Umfrage konfiguriert ist, die die Kalibrierungsdaten im Vergleich zum vorherigen Satz von Kalibrierungsdaten bewertet.

10. System nach Anspruch 1, wobei die Konfiguration des Steuersystems eines oder mehrere von einer vom Steuersystem bereitgestellten Beleuchtungsquelle, einem Typ des Bildprozessors und einer Software-Release-Version des Steuersystems umfasst.

11. System nach Anspruch 10, wobei die Kalibrierungsdaten zum Verbessern eines für den Typ von Prozedur, in dem das Bildaufnahmegerät verwendet werden soll, signifikantes Bildmerkmal erzeugt werden,
wobei das Bildmerkmal optional eines oder mehrere von Fokusniveau, Schärfe, Farbtreue und Farbgenauigkeit ist.

12. System nach Anspruch 10, wobei der Kalibrierungsprozessor ferner zum Abrufen eines während der Prozedur durch das Bildaufnahmegerät (500) aufgenommenen Betriebsbildes aus dem Steuersystem und zum Justieren der Kalibrierungsdaten auf der Basis der Betriebsbilder konfiguriert ist,
wobei ein Luminanzwert des Betriebsbildes optional mit einem erwarteten Luminanzwert verglichen wird, der auf der Basis eines bekannten Abstands zu einem in dem Betriebsbild erfassten Objekt und einer bekannten Leistungsabgabe an eine das in dem Betriebsbild erfasste Objekt beleuchtende Beleuchtungsquelle bestimmt wird,
wobei optional ein Farbraum des Betriebsbildes mit einem erwarteten Farbraum des Bildaufnahmegeräts verglichen wird, um festzustellen, ob eine Mitte des Farbraums von einer Mitte des erwarteten Farbraums versetzt ist,
wobei optional eine stereoskopische Ausrichtung des Betriebsbildes ausgewertet wird, um eine Fehlausrichtung mit einem entsprechenden Betriebsbild zu identifizieren.

13. System nach einem der Ansprüche 1-5, wobei das Steuersystem ferner einen Kalibrierungs-Cache-Speicher umfasst, der zum Speichern der aus der über das Netzwerk zugänglichen Datenbank (602) abgerufenen Kalibrierungsdaten konfiguriert ist,
wobei das Steuersystem optional ferner zum Speichern von Kalibrierungsdaten von Bildaufnahmegeräten konfiguriert ist, die bekanntlich an einen mit einem Ort des Steuersystems assoziierten Ort geliefert wurden,
wobei das Steuersystem optional ferner zum Speichern von Kalibrierungsdaten einer vorbestimmten Anzahl von zuvor verwendeten Bildaufnahmegeräten (500) konfiguriert ist,
wobei das Steuerungssystem optional ferner zum Speichern von Standardkalibrierungsdaten für einen Typ des Bildaufnahmegeräts (500) konfiguriert ist,
wobei das Steuersystem optional ferner zum Vergleichen eines gecachten Satzes von Kalibrierungsdaten für das Bildaufnahmegerät mit den in der über das Netzwerk zugänglichen Datenbank gespeicherten Kalibrierungsdaten konfiguriert ist, bevor die Kalibrierungsdaten aus der über das Netzwerk zugänglichen Datenbank abgerufen werden.

14. Verfahren, das Folgendes umfasst:
Empfangen, durch einen Kalibrierungsprozessor in Kommunikation mit einer über ein Netzwerk zugänglichen Datenbank (602), von durch ein Bildaufnahmegerät (500) aufgenommenen Kalibrierungsbildern von einem Steuersystem (24) für ein Bildaufnahmegerät (500);
Erzeugen, durch den Kalibrierungsprozessor, von Kalibrierungsdaten als Reaktion auf eine Änderung in einer Konfiguration des Steuersystems (24), wobei die Kalibrierungsdaten auf der Basis der Anwendung eines Kalibrierungsalgorithmus auf eines oder mehrere der Kalibrierungsbilder erzeugt werden, wobei der Kalibrierungsalgorithmus auf der Basis von einem oder mehreren von einem Typ von Prozedur, in dem das Bildaufnahmegerät (500) verwendet werden soll, der Konfiguration des Steuersystems (24) und der Verfügbarkeit einer anderen Kalibrierungsmethodik zum Berechnen eines oder mehrerer Parameter der Kalibrierungsdaten ausgewählt wird;
Speichern der Kalibrierungsdaten für das Bildaufnahmegerät (500) in der über ein Netzwerk zugänglichen Datenbank (602);
Abrufen, durch das Steuersystem (24), der Kalibrierungsdaten aus der über ein Netzwerk zugänglichen Datenbank über eine Netzwerkverbindung;
Empfangen, durch das Steuersystem, von von dem Bildaufnahmegerät (500) aufgenommenen zweiten Bildern; und
Verarbeiten, durch einen Bildprozessor des Steuersystems (24), der empfangenen zweiten Bilder auf der Basis der abgerufenen Kalibrierungsdaten.

## Revendications

1. Système, comprenant :
une base de données accessible par réseau (602) configurée pour stocker des données d'étalonnage pour un dispositif de capture d'images (500) ;
un système de commande (24) comprenant un processeur d'images configuré pour recevoir des images capturées par le dispositif de capture d'images, le processeur d'images étant en outre configuré pour récupérer les données d'étalonnage dans la base de données accessible par réseau par l'intermédiaire d'une connexion de réseau, et le processeur d'images étant en outre configuré pour traiter les images reçues sur la base des données d'étalonnage récupérées ; et
un processeur d'étalonnage en communication avec la base de données accessible par réseau (602), le processeur d'étalonnage étant configuré pour recevoir des images d'étalonnage capturées par le dispositif de capture d'images (500) et générer les données d'étalonnage en réponse à une modification d'une configuration du système de commande (24), les données d'étalonnage étant générées sur la base de l'application d'un algorithme d'étalonnage à une ou plusieurs des images d'étalonnage, l'algorithme d'étalonnage étant sélectionné sur la base d'un ou plusieurs éléments parmi : un type de procédure au cours de laquelle le dispositif de capture d'images (500) doit être utilisé, la configuration du système de commande (24) et la disponibilité d'une méthodologie d'étalonnage différente pour calculer un ou plusieurs paramètres des données d'étalonnage.

2. Système selon la revendication 1, comprenant en outre :
un outil comprenant le dispositif de capture d'images (500) et couplé électriquement au système de commande (24), l'outil ayant un identifiant unique ;
de préférence, les données d'étalonnage étant indexées avec le dispositif de capture d'images dans la base de données accessible par réseau, sur la base de l'identifiant unique ;
plus préférablement, l'identifiant unique étant conservé par l'outil, et le processeur d'images étant en outre configuré pour obtenir l'identifiant unique auprès de l'outil.

3. Système selon la revendication 2, l'identifiant unique étant conservé par l'outil sur un dispositif de mémoire de l'outil, sur une étiquette sans fil de l'outil ou sur un indicateur visuel sur une surface de l'outil.

4. Système selon la revendication 3, le processeur d'images comprenant en outre un scanner optique configuré pour lire l'indicateur visuel de la surface de l'outil afin d'obtenir l'identifiant unique de l'outil.

5. Système selon la revendication 4, le scanner optique étant situé dans un réceptacle d'accouplement configuré pour coupler l'outil au système de commande ;
de préférence, le scanner optique étant une caméra ou un lecteur de codes-barres.

6. Système selon l'une quelconque des revendications 3 à 5, l'indicateur visuel étant imprimé, collé, étiqueté, gaufré ou estampé sur la surface de l'outil ;
éventuellement, l'indicateur visuel étant un numéro de série alphanumérique, un code-barres linéaire qui code l'identifiant unique, un code-barres bidimensionnel qui code l'identifiant unique ou une image codée avec l'identifiant unique.

7. Système selon l'une quelconque des revendications 2 à 5, l'outil comprenant un endoscope (28) et le dispositif de capture d'images comprenant une paire de capteurs d'images configurés pour capturer des données d'images stéréoscopiques d'un site chirurgical pendant une intervention chirurgicale.

8. Système selon l'une quelconque des revendications 1 à 5, les données d'étalonnage comprenant des données permettant de traiter les images reçues afin de corriger les images reçues sur la base d'un ou plusieurs éléments parmi une couleur de lumière émise par un dispositif d'éclairage configuré pour éclairer une scène capturée par les images reçues, des images d'étalonnage capturées par le dispositif de capture d'images, des distorsions optiques introduites par un système optique du dispositif de capture d'images, un étalonnage d'image stéréoscopique, un mappage d'espace colorimétrique et une caractéristique de capteur d'images du dispositif de capture d'images.

9. Système selon la revendication 1, les images d'étalonnage étant stockées dans la base de données accessible par réseau (602) avec les données d'étalonnage,
éventuellement, les données d'étalonnage étant différentes d'un ensemble antérieur de données d'étalonnage pour le dispositif de capture d'images, l'ensemble antérieur de données d'étalonnage étant généré par le processeur d'étalonnage sur la base de l'application d'un algorithme d'étalonnage différent à une ou plusieurs des images d'étalonnage,
éventuellement, le processeur d'étalonnage étant en outre configuré pour recevoir un sondage de rétroaction évaluant les données d'étalonnage par comparaison avec l'ensemble antérieur de données d'étalonnage.

10. Système selon la revendication 1, la configuration du système de commande comprenant un ou plusieurs éléments parmi une source d'éclairage fournie par le système de commande, un type du processeur d'images et une version logicielle majeure du système de commande.

11. Système selon la revendication 10, les données d'étalonnage étant générées pour améliorer une caractéristique d'image significative pour le type de procédure au cours de laquelle le dispositif de capture d'images doit être utilisé,
éventuellement, la caractéristique d'image étant une ou plusieurs caractéristiques parmi un niveau de mise au point, une netteté, une fidélité des couleurs et une précision des couleurs.

12. Système selon la revendication 10, le processeur d'étalonnage étant en outre configuré pour récupérer une image fonctionnelle capturée par le dispositif de capture d'images (500) pendant la procédure à partir du système de commande et pour régler les données d'étalonnage sur la base des images fonctionnelles,
éventuellement, une valeur de luminance de l'image fonctionnelle étant comparée à une valeur de luminance attendue déterminée sur la base d'une distance connue par rapport à un objet capturé dans l'image fonctionnelle et d'une puissance connue délivrée en sortie à une source d'éclairage qui éclaire l'objet capturé dans l'image fonctionnelle,
éventuellement, un espace colorimétrique de l'image fonctionnelle étant comparé à un espace colorimétrique attendu du dispositif de capture d'images afin de déterminer si un centre de l'espace colorimétrique est décalé par rapport à un centre de l'espace colorimétrique attendu,
éventuellement, un alignement stéréoscopique de l'image fonctionnelle étant évalué afin d'identifier un désalignement par rapport à une image fonctionnelle correspondante.

13. Système selon l'une quelconque des revendications 1 à 5, le système de commande comprenant en outre une mémoire cache d'étalonnage configurée pour stocker les données d'étalonnage récupérées dans la base de données accessible par réseau (602),
éventuellement, le système de commande étant en outre configuré pour stocker des données d'étalonnage de dispositifs de capture d'images connus pour avoir été expédiés vers une localisation associée à une localisation du système de commande,
éventuellement, le système de commande étant en outre configuré pour stocker des données d'étalonnage d'un nombre prédéterminé de dispositifs de capture d'images (500) utilisés précédemment,
éventuellement, le système de commande étant en outre configuré pour stocker des données d'étalonnage par défaut pour un type du dispositif de capture d'images (500),
éventuellement, le système de commande étant en outre configuré pour comparer un ensemble mis en cache de données d'étalonnage pour le dispositif de capture d'images aux données d'étalonnage stockées dans la base de données accessible par réseau avant de récupérer les données d'étalonnage dans la base de données accessible par réseau.

14. Procédé, comprenant les étapes consistant à :
recevoir, par un processeur d'étalonnage en communication avec une base de données accessible par réseau (602), en provenance d'un système de commande (24) pour un dispositif de capture d'images (500), des images d'étalonnage capturées par le dispositif de capture d'images (500) ;
générer, parle processeur d'étalonnage, des données d'étalonnage en réponse à une modification d'une configuration du système de commande (24), les données d'étalonnage étant générées sur la base de l'application d'un algorithme d'étalonnage à une ou plusieurs des images d'étalonnage, l'algorithme d'étalonnage étant sélectionné sur la base d'un ou plusieurs éléments parmi : un type de procédure au cours de laquelle le dispositif de capture d'images (500) doit être utilisé, la configuration du système de commande (24) et la disponibilité d'une méthodologie d'étalonnage différente pour calculer un ou plusieurs paramètres des données d'étalonnage ;
stocker les données d'étalonnage pour le dispositif de capture d'images (500) dans la base de données accessible par réseau (602) ;
récupérer, par le système de commande (24), les données d'étalonnage dans la base de données accessible par réseau par l'intermédiaire d'une connexion de réseau ;
recevoir, par le système de commande, des secondes images capturées par le dispositif de capture d'images (500) ; et
traiter, par un processeur d'images du système de commande (24), les secondes images reçues, sur la base des données d'étalonnage récupérées.
